# EUROPEAN PATENT SPECIFICATION

(11) **EP 1 384 789 B1**
(45) Date of publication and mention of the grant of the patent: **20.09.2006**
(21) Application number: 03016378.6
(22) Date of filing: 19.07.2003
(51) Int. Cl.: C12Q 1/68

(54) **Fluorescent hybridization probes with reduced background**
Fluoreszenzmarkierten Hybridisierungssonden mit reduzierter Hintergrundfluoreszenz
Sondes d'hybridation fluorescentes ayant un bruit de fond réduit

(30) Priority: 23.07.2002 EP 02016265
(43) Date of publication of application: 28.01.2004
(73) Proprietor: Roche Diagnostics GmbH, 68305 Mannheim (DE); F.HOFFMANN-LA ROCHE AG, 4070 Basel (CH)
(72) Inventor: Heindl, Dieter, Dr., 82396 Paehl (DE); Josel, Hans-Peter, Dr., 82362 Weilheim (DE); Sagner, Gregor, Dr., 82377 Penzberg (DE); Mayr, Jutta, Dr., 82061 Neuried (DE)

(56) References cited:
- WO-A-97/46707
- US-A- 6 037 130
- OKAMURA Y ET AL: "Double-labeled donor probe can enhance the signal of fluorescence resonance energy transfer (FRET) in detection of nucleic acid hybridization." NUCLEIC ACIDS RESEARCH. ENGLAND 15 DEC 2000, vol. 28, no. 24, 15 December 2000 (2000-12-15), page E107 XP002215063 ISSN: 1362-4962
- SEIDEL C A ET AL: "NUCLEOBASE-SPECIFIC QUENCHING OF FLUORESCENT DYES. 1. NUCLEOBASE ONE-ELECTRON REDOX POTENTIALS AND THEIR CORRELATION WITH STATIC AND DYNAMIC QUENCHING EFFICIENCIES" JOURNAL OF PHYSICAL CHEMISTRY, AMERICAN CHEMICAL SOCIETY, US, vol. 100, 1996, pages 5541-5553, XP000925372 ISSN: 0022-3654
- DIDENKO V V: "DNA PROBES USING FLUORESCENCE RESONANCE ENERGY TRANSFER (FRET): DESIGNS AND APPLICATIONS" BIOTECHNIQUES, EATON PUBLISHING, NATICK, US, vol. 5, no. 31, November 2001 (2001-11), pages 1106-1121, XP001082961 ISSN: 0736-6205
- FRUTOS ANTHONY G ET AL: "Method for detection of single-base mismatches using bimolecular beacons." JOURNAL OF THE AMERICAN CHEMICAL SOCIETY, vol. 124, no. 11, 20 March 2002 (2002-03-20), pages 2396-2397, XP001098816 March 20, 2002 ISSN: 0002-7863
- TYAGI S ET AL: "Multicolor molecular beacons for allele discrimination." NATURE BIOTECHNOLOGY. UNITED STATES JAN 1998, vol. 16, no. 1, January 1998 (1998-01), pages 49-53, XP002143901 ISSN: 1087-0156
- BERGSTROM DONALD E ET AL: "Comparison of the base pairing properties of a series of nitroazole nucleobase analogs in the oligodeoxyribonucleotide sequence 5'-d(CGCXAATTYGCG)-3'." NUCLEIC ACIDS RESEARCH, vol. 25, no. 10, 1997, pages 1935-1942, XP002215064 ISSN: 0305-1048
- NAZARENKO IRINA ET AL: "Effect of primary and secondary structure of oligodeoxyribonucleotides on the fluorescent properties of conjugated dyes." NUCLEIC ACIDS RESEARCH. ENGLAND 1 MAY 2002, vol. 30, no. 9, 1 May 2002 (2002-05-01), pages 2089-2195, XP002215065 ISSN: 1362-4962
- TYAGI SANJAY ET AL: "Wavelength-shifting molecular beacons." NATURE BIOTECHNOLOGY, vol. 18, no. 11, November 2000 (2000-11), pages 1191-1196, XP002215066 ISSN: 1087-0156

### SEQUENCE LISTING

<110> Roche Diagnostics GmbH
   F. Hoffmann-La Roche AG
<120> Fluorescent hybridization probes with reduced background
<130> 21329 EP
<140>
   <141>
<160> 9
<170> PatentIn Ver. 2.1
<210> 1
   <211> 22
   <212> DNA
   <213> Artificial Sequence
<220>
   <223> Description of Artificial Sequence:primer
<400> 1
   gagagacatc gcctctgggc ta 22
<210> 2
   <211> 20
   <212> DNA
   <213> Artificial Sequence
<220>
   <223> Description of Artificial Sequence:primer
<400> 2
   tgttatcaca ctggtgctaa 20
<210> 3
   <211> 23
   <212> DNA
   <213> Artificial Sequence
<220>
   <223> Description of Artificial Sequence:primer
<400> 3
   aatacctgta ttcctcgcct gtc 23
<210> 4
   <211> 36
   <212> DNA
   <213> Artificial Sequence
<220>
   <223> Description of Artificial Sequence:primer
<400> 4
   agggatctgc tcttacagat tagaagtagt cctatt 36
<210> 5
   <211> 21
   <212> DNA
   <213> Artificial Sequence
<220>
   <223> Description of Artificial Sequence:primer
<400> 5
   tgtgaggttg tcatgggcaa c 21
<210> 6
   <211> 26
   <212> DNA
   <213> Artificial Sequence
<220>
   <223> Description of Artificial Sequence:primer
<400> 6
   aggtaacgaa actgattaag agccac 26
<210> 7
   <211> 28
   <212> DNA
   <213> Artificial Sequence
<220>
   <223> Description of Artificial Sequence:primer
<400> 7
   cggtctgttc gagaagtcac aggctacg 28
<210> 8
   <211> 25
   <212> DNA
   <213> Artificial Sequence
<220>
   <223> Description of Artificial Sequence:primer
<400> 8
   gagcacaacc gggacctctc cttcc 25
<210> 9
   <211> 26
   <212> DNA
   <213> Artificial Sequence
<220>
   <223> Description of Artificial Sequence:primer
<400> 9
   tgagcacaac cgggacctct ccttcg 26

## Description

The invention originates from the field of real time PCR. More specifically, the invention is directed to an improved pair of FRET Hybridization probes.

### Prior art Background

In kinetic real-time PCR, the formation of PCR products is monitored in each cycle of the PCR. The amplification is usually measured in thermocyclers which have additional devices for measuring fluorescence signals during the amplification reaction. A typical example of this is the Roche Diagnostics LightCycler (Cat. No. 2 0110468). The amplification products are for example detected by means of fluorescent labeled hybridization probes which only emit fluorescence signals when they are bound to the target nucleic acid or in certain cases also by means of fluorescent dyes that bind to double-stranded DNA. A defined signal threshold is determined for all reactions to be analyzed and the number of cycles Cp required to reach this threshold value is determined for the target nucleic acid as well as for the reference nucleic acids such as the standard or housekeeping gene. The absolute or relative copy numbers of the target molecule can be determined on the basis of the Cp values obtained for the target nucleic acid and the reference nucleic acid (97/46707; WO 97/46712; WO 97/46714).

The FRET real time PCR test format is characterized by two single-stranded hybridization probes which are used simultaneously and are complementary to adjacent sites of the same strand of the amplified target nucleic acid. Both probes are labeled with different fluorescent components. When excited with light of a suitable wavelength, a first component transfers the absorbed energy to the second component according to the principle of fluorescence resonance energy transfer such that a fluorescence emission of the second component can be measured when both hybridization probes bind to adjacent positions of the target molecule to be detected. (Alternatively, fluorescence decrease of the FRET donor component may be monitored.) Among all detection formats known in the art, this "FRET-hybridization probe format" has been proven to be highly sensitive, exact and reliable. The design of appropriate FRET hybridization probe sequences may sometimes be limited by the special characteristics of the target nucleic acid sequence to be detected.

Alternatively, it is also possible to use a fluorescent-labeled primer and only one labeled oligonucleotide probe (Bernard, P. S., et al., Anal Biochem 255 (1998) 101-7).

Another application of FRET hybridization probes is melting curve analysis. In such an assay, the target nucleic acid is amplified first in a typical PCR reaction with suitable amplification primers The hybridization probes may already be present during the amplification reaction or added subsequently. After completion of the PCR-reaction, the temperature of the sample is constitutively increased, and fluorescence is detected as long as the hybridization probe was bound to the target DNA. At melting temperature, the hybridization probes are released from their target, and the fluorescent signal is decreasing immediately down to the background level. This decrease was monitored with an appropriate fluorescence versus temperature-time plot, a first derivative fluorescence versus temperature plot, an exact temperature value can be determined, at which the fluorescence decrease was observed.

It has been observed however, that depending on the type of fluorescent dye which is used, in most cases there exists a reasonable background fluorescence. This results in a limited dynamic range of the assay, especially, if the fluorescent signals are normalized for background fluorescence by any method known in the art.

It is known from literature that double or multiple labeled Fluorescein donor probes enhance the FRET process such that an increased emission of the FRET acceptor compound is achieved. However, the efficiency of this alternative strongly depends on the spatial orientation of the dyes with respect to the donor probe (Okamura, Y., et al., Nucleic Acids Res 28 (2000) E107). Moreover, multiple fluorescent donor oligonucleotides are difficult to purify and the monomeric donor phosphoramidite building blocks for oligonucleotides are expensive starting materials. In addition, this principle is not universal and is limited to base stable dyes where the phosphoramidite approach is feasible.

Thus there is a need for FRET Hybridization probes which provide improved signal intensity and a fairly broad dynamic range of detection, independent from the type of labeling dyes which are used.

### Brief description of the invention

This problem is resolved by FRET hybridization probes according to the present invention
wherein the FRET donor probe itself is carrying a chemical moiety capable of quenching the fluorescent donor moiety, said chemical moiety being a Nitroindole.

More precisely, the new invention is directed to a pair of FRET hybridization probes hybridizing adjacently to each other on a target nucleic acid. Said pair of FRET hybridization probes consists of a first oligonucleotide carrying a FRET donor entity and a second oligonucleotide carrying a FRET acceptor entity, wherein the first oligonucleotide is carrying at least one second entity, said second entity being Nitroindole which is capable of quenching fluorescence emission of said donor fluorescent entity.

Preferably, the nucleotide residue of the first oligonucleotide carrying the donor fluorescence entity is also carrying the second entity with the quenching activity.

It is also within the scope of the invention, if one member of said pair of FRET hybridization probes may serve as a primer in a nucleic acid amplification reaction such as the polymerase chain reaction (PCR).

Moreover, the present invention is directed to a method of using the inventive pair of FRET hybridization probes disclosed above, wherein fluorescence emission of either the donor fluorescent entity or emission of the acceptor fluorescent entity is monitored in Real Time. In a specific embodiment, fluorescence emission of said FRET donor entity is monitored in a first detector channel and fluorescence emission of said FRET acceptor entity is monitored in a second detector channel, such that the fluorescence emission of said FRET acceptor entity can be normalized by the fluorescence emission of said FRET donor entity.

### Description of the Figures

Fig. 1
   F2/F1-fluorescence versus cycle number plot of the real time PCR experiment disclosed in example 3.
   +N/-N: with/without a Nitroindole group at the FRET donor
   a) 10⁸ copies, b) 10³ copies of target DNA
Fig. 2
   1^{st} derivative of F2/F1-fluorescence versus temperature plot showing the melting curve analysis of the experiment disclosed in example 4.
   .+N/-N: with/without a Nitroindole group at the FRET donor
   a) 10⁸ copies, b) 10³ copies of target DNA
Fig. 3
   F2/F1 fluorescence versus cycle number plot of the real time PCR experiment disclosed in example 5.

The upper curve corresponds to detection with using Fluorescein labeled probe carrying an additional G according to SEQ ID NO: 9, whereas the lower curve corresponds to detection with a Fluorescein labeled probe as known in the art according to SEQ ID NO: 8.

### Detailed description of the invention

In real time PCR using FRET hybridization probes, fluorescence is monitored as a measure for the amount of amplification product which has been synthesized. For obtaining reliable and quantitative data, it has been proven to be advantageous to perform a normalization of the absolute signal for background fluorescence signaling. In order to achieve this, fluorescence emission of the FRET acceptor is monitored in a first detector channel and fluorescence emission of the FRET donor is monitored in a second detector channel. As a consequence, fluorescence emission of the FRET acceptor can be normalized by means of dividing the obtained value by the fluorescence emission of said FRET donor.

However, dependent on the fluorescent dyes which are actually used for creating a FRET pair, the normalized values are sometimes far from being optimal. This results in a low dynamic range of a respective assay. In this context, the inventors observed that this effect is predominantly due to background signaling of the FRET donor moiety. Due to the general idea of the present invention, this problem may be overcome by an appropriate quenching of the FRET donor signal.

In order to achieve this, FRET donor moieties are quenched by bringing one or more quencher molecules in close vicinity to the FRET donor moiety. According to the invention, this is done in such a way that the oligonucleotide carrying the FRET donor moiety in addition carries Nitroindole as a second entity which is capable of quenching the FRET donor moiety itself. Preferably, the residue of the nucleotide residue carrying the donor fluorescence entity is also carrying the second entity with the quenching activity.

The inventive method improves the options to design a FRET assay and improves signal dynamics of the FRET detection process, since the interfering emission of the FRET donor is significantly reduced. This is especially important, when the spectrum of the FRET donor emission overlaps with the spectrum of the FRET acceptor emission at least partially.

Surprisingly, despite the presence of a quencher moiety close to FRET donor moiety, the FRET donor is still able to act as a FRET partner. Even more surprisingly, compared to the FRET donor fluorescence, the FRET donor energy transfer activity is less reduced under these conditions for all quenchers tested by the inventors so far. Thus, the introduction of a Nitroindole moiety according to the invention significantly reduces background fluorescence of the FRET donor, but does not affect the efficiency of the FRET process itself.

In a first aspect, the present invention is directed to a pair of FRET hybridization probes consisting of a first oligonucleotide carrying at least a FRET donor entity and a second oligonucleotide carrying at least a FRET acceptor entity. Said first oligonucleotide carrying the donor fluorescent entity is carrying at least one second entity, which is a Nitroindole, which is capable of quenching fluorescence emission of said donor fluorescent entity.

The position of the nucleotide residue within the first oligonucleotide may be chosen arbitrarily as long as it results in a quenching effect with regard to emission of the FRET donor entity. Preferably, however, the two residues carrying the FRET donor entity and the quencher entity are in close spatial vicinity to each other. For example, these two residues may be adjacent residues. Most preferably, the same residue is labeled with both the fluorescence donor entity and the Nitroindole entity.

Also preferably, one oligonucleotide carrying the first FRET entity is labeled at its 3' terminal residue and the second oligonucleotide carrying the second FRET entity is labeled at its 5' terminal residue such that when both oligonucleotides are hybridized to the target nucleic acid, the fluorescent labels of both FRET entities are brought in close vicinity to each other due to the fact that said terminal residues are base pairing to adjacent residues in the target nucleic acid or at least to residues which are only separated by one or two further residues. In this context, the oligonucleotide which carries the FRET donor moiety together with the inventive quencher compound may be chosen arbitrarily, since these elements can be introduced on both, either the 5' or the 3' end of an oligonucleotide.

In a second aspect, the present invention is directed to a set of at least 3 oligonucleotides, characterized in that a first oligonucleotide and a second oligonucleotide are capable of acting as a pair of amplification primers for a template dependent nucleic acid amplification reaction. Said first oligonucleotide and said third oligonucleotide are each labeled with at least one corresponding member of a FRET pair consisting of a FRET donor entity and a FRET acceptor entity, wherein the oligonucleotide carrying the FRET donor entity is carrying at least one second entity, said second entity being a Nitroindole which is capable of quenching fluorescence emission of said donor fluorescent entity. Again, the two residues of the first oligonucleotide carrying the FRET donor entity and the quencher entity are preferably in close spatial vicinity to each other. For example, these two residues may be adjacent residues. Most preferably, the same residue is labeled with both the fluorescence donor entity and the Nitroindole quenching entity.

It is important that upon hybridization of said third oligonucleotide to the amplified target nucleic acid, the labels of said first oligonucleotide and said third nucleotide are brought in close vicinity to each other such that they are separated by no, one, two or only a few nucleotide residues. This may for example be achieved by a primer/probe design, characterized in that said first oligonucleotide and a third oligonucleotide are partially complementary to each other. In this regard, the term "partially" need to be understood as comprising embodiments, wherein complementarity is restricted to one, two, three or only a few oligonucleotides.

Preferably, the oligonucleotide which serves as a primer and at the same time is labeled in order to be able to participate in the FRET, is preferentially labeled at an internal nucleotide residue, because a 3' terminal labeling would inhibit elongation by a template dependent polymerase and thus result in lack of amplification product. On the other hand, a 5' terminal labeling is not applicable as well, since a label at this position would require an oligonucleotide probe which would be almost complementary to said first oligonucleotide. This again would lead to problems, during the amplification process because the first oligonucleotide and the oligonucleotide probe could already form hybrids prior to the amplification reaction and result in an increased background fluorescence.

Preferably, the third oligonucleotide which is used as hybridization probe is labeled at its 5' terminal residue or at its 3' terminal residue with both the FRET donor moiety and the Nitroindole quencher according to the invention. Upon hybridization with the amplified target nucleic acid, the fluorescent labels of both FRET entities are brought into close vicinity to each other such that the two labeled nucleotide residues either form base pairs with each other or at least with adjacent residues.

In a third aspect, the present invention is directed to compositions comprising the FRET oligonucleotides disclosed above. More precisely, a composition according to the invention comprises a nucleic acid sample and a pair of hybridization probes characterized in that this pair consists of a first oligonucleotide carrying a FRET donor entity and a second oligonucleotide carrying a FRET acceptor entity, wherein said first oligonucleotide carrying the donor fluorescent entity is carrying at least one second entity, said second entity being a Nitroindole which is capable of quenching fluorescence emission of said donor fluorescent entity.

Alternatively, the composition according to the invention may comprise a set of at least 3 oligonucleotides, characterized in that a first oligonucleotide and a second oligonucleotide are capable of acting as a pair of amplification primers for a template dependent nucleic acid amplification reaction. The first oligonucleotide and the third oligonucleotide are each labeled with one corresponding member of a FRET pair consisting of a FRET donor entity and a FRET acceptor entity, wherein the oligonucleotide carrying the FRET donor entity is carrying at least one second entity, said second entity being a Nitroindole which is capable of quenching fluorescence emission of said donor fluorescent entity. Said first oligonucleotide and said third oligonucleotide may preferentially be partially complementary to each other over a string of one, two, three, or a several few residues.

The present invention is also directed to various methods and applications of using the inventive oligonucleotide pairs and compositions disclosed above.

More precisely, the present invention is directed to a method for qualitative or quantitative detection of a nucleic acid sequence in a nucleic acid sample, wherein said nucleic acid sample is being hybridized with a pair of FRET hybridization probes consisting of a first oligonucleotide carrying at least a FRET donor entity and a second oligonucleotide carrying at least a FRET acceptor entity. Said first oligonucleotide carrying the donor fluorescent entity is carrying at least one second entity, which is a Nitroindole capable of quenching fluorescence emission of said donor fluorescent entity.

In one embodiment, such a method may be a typical hybridization assay. The hybridization may take place either in solution, or alternatively, either the target nucleic acid or one member of the pair of FRET hybridization probes already by immobilized on a solid support. The solid support itself, for example can be a hybridization membrane, a magnet glass bead, a microarray for immobilizing nucleic acids or any other material known in the art.

In another, highly preferred embodiment, a part of said nucleic acid present in the sample is being subjected to a nucleic acid amplification reaction prior or during the hybridization procedure, for example, a polymerase chain reaction (PCR). As a prerequisite, the target nucleic acid comprises a sequence substantially complementary to the sequence of the used hybridization probes. In other words, the used hybridization probes need to hybridize specifically to the target nucleic acid.

Alternatively, in case the target nucleic acid is being amplified, a set of at least 3 different oligonucleotides is used. The set is characterized in that a first oligonucleotide and a second oligonucleotide are capable of acting as a pair of amplification primers for a template dependent nucleic acid amplification reaction. Said first oligonucleotide and said third oligonucleotide are each labeled with at least one corresponding member of a FRET pair consisting of a FRET donor entity and a FRET acceptor entity, wherein the oligonucleotide carrying the FRET donor entity is carrying at least one second entity, said second entity being a Nitroindole which is capable of quenching fluorescence emission of said donor fluorescent entity.

In addition, the methods disclosed above allow for a monitoring of the amplification reaction in real time by means of monitoring fluorescence emission of either the donor fluorescent entity or emission of the acceptor fluorescent entity during the amplification reaction itself. Thus real time monitoring allows the generation of kinetic data and facilitates quantitative analysis.

A low background of the FRET donor entity is especially important in cases wherein fluorescence emission of the FRET acceptor entity is normalized by the fluorescence emission of said FRET acceptor entity. Normalization is usually performed by means of dividing the value monitored for fluorescence emission of the FRET acceptor by the value monitored for the fluorescence emission of said FRET acceptor. Consequently, a low background results in a high normalized score.

In this context, the new invention is also directed to a method for qualitative or quantitative detection of a nucleic acid sequence in a nucleic acid sample, characterized in that fluorescence emission of the FRET donor entity is monitored in a first detector channel and fluorescence emission of said FRET acceptor entity is monitored in a second detector channel, further characterized in that the fluorescence emission of said FRET acceptor entity is normalized by the fluorescence emission of said FRET donor entity.

In a further aspect, the present invention is directed to the usage of the FRET hybridization probes disclosed above for melting curve analysis, wherein monitoring of the dissociation of a complex between a target nucleic acid and a hybridization probe allows for the detection of small sequence variants such as single nucleotide polymorphisms.

More precisely, in one aspect, the invention is directed to a method, wherein first, a ternary hybrid complex between the target nucleic acid and the two hybridization probes is formed. Subsequently, the temperature is increased and the thermal dissociation of the ternary complex is determined by means of monitoring fluorescence in real time. In other words, the new invention is also directed to a method for the determination of the melting profile of a hybrid consisting of a target nucleic acid and a pair of FRET hybridization probes as disclosed above, characterized in that the fluorescence emission is determined as a function of temperature.

Alternatively, it is also possible to determine a melting profile of a hybrid consisting of a target nucleic acid and only one oligonucleotide in cases, wherein the first partner of a FRET pair has been introduced into the amplification product upon usage of an appropriately labeled primer, and the second partner of a FRET pair is being provided by an appropriately labeled oligonucleotide, such that thermal dissociation of a binary hybrid complex between the target nucleic acid and the labeled oligonucleotide functioning as a hybridization probe can be monitored.

Also for performing melting curve analysis, according to the invention it has been proven to be advantageous, if fluorescence emission of the FRET acceptor moiety is monitored in a first detector channel and fluorescence emission of the FRET donor moiety is monitored in a second detector channel, such that the value obtained for fluorescence emission of said FRET acceptor entity can normalized by the fluorescence emission of said FRET donor entity.

In a last aspect, the present invention is directed to a kit comprising a pair of hybridization probes according to the invention.

Such a kit may comprise a set of FRET hybridization probes according to the invention as disclosed above. In addition, it may also contain oligonucleotides capable of acting as a primer pair for a nucleic acid amplification reaction.

Precisely, the kit comprises a pair of FRET hybridization probes consisting of a first oligonucleotide carrying a FRET donor entity and a second oligonucleotide carrying a FRET acceptor entity, wherein said first oligonucleotide carrying the donor fluorescent entity is carrying at least one second entity, said second entity being a Nitroindole which is capable of quenching fluorescence emission of said donor fluorescent entity. Preferably, the two residues carrying the FRET donor entity and the quencher entity are in close spatial vicinity to each other. For example, these two residues may be adjacent residues. Most preferably, the same residue is labeled with both the fluorescence donor entity and the quenching entity.

Alternatively, the kit comprises a set of at least 3 oligonucleotides, characterized in that a first oligonucleotide and a second oligonucleotide are capable of acting as a pair of amplification primers for a template dependent nucleic acid amplification reaction further characterized in that said first oligonucleotide and said third oligonucleotide are each labeled with one corresponding member of a FRET pair consisting of a FRET donor entity and a FRET acceptor entity, wherein the oligonucleotide carrying the FRET donor entity is carrying at least one second entity, said second entity being a Nitroindole which is capable of quenching fluorescence emission of said donor fluorescent entity. Again, the two residues of the first oligonucleotide carrying the FRET donor entity and the quencher entity are preferably in close spatial vicinity to each other. For example, these two residues may be adjacent residues. Most preferably, the same residue is labeled with both the fluorescence donor entity and the Nitroindole quenching entity.

In addition, a kit according to the present invention may contain at least one additional component such as a nucleic acid polymerase, deoxynucleoside triphosphates or respective analogues and an appropriate buffer which may be used for a template dependent nucleic acid amplification reaction such as PCR. Furthermore, the kit may also comprise software tools such as compact discs carrying computer programs for quantitative analysis of relative or absolute nucleic acid quantification experiments.

### Example 1

### Preparation of PCR primers and probes

Primers were synthesized on a 1 µmol scale on ABI 394 synthesizer using commercially available standard phosphoramidites ( DMTr ibu G, DMTr bzA; DMTr bz C and DMTr T) and the corresponding CPG support. The chemicals for standard synthesis were obtained from GlenResearch. Removal of the oligonucleotides from the solid support and deprotection was carried out with 33 % NH₃ for 8h at 55 °C. Synthesis was performed in the trityl on modus. Purification was done on a RP 18 Oligo R3 4.6 x 50 mm column from Perseptive Biosystems. Buffer A: 0.1M Triethylammonium acetate in water pH 7.0 /MeCN 95:5 buffer B: MeCN. gradient 3 min 20 % B; 12 min 12- 40 % B flow rate 1 ml/min detection 260 nm. Subsequently, the concentrated oligonucleotide solution was treated for 5 min with 80 % Acetic acid at room temperature in order to remove the 5' DMTr protecting group. Afterwards, oligonucleotides were desalted with a RP 18 column and lyophilyzed in a Speed Vac.

### Example 2

### Preparation of a fluorescently labeled probes and probes carrying a Nitroindole moiety

Oligonucleotide synthesis was performed in the 1 µmol range. Commercially available standard phosphoramidites ( DMTr ibu G, DMTr bzA; DMTr bz C and DMTr T) and chemicals for standard synthesis were obtained from Glen Research. The 5' fluorescein was introduced by using commercially available 5' Fluorescein Phosphoramidite (Glen Research (cat no. 10-1916-90). Nitroindole was introduced during synthesis by using commercially available 5Nitro indole Phosphoramidite (Glen Research cat. No. 10-1044-90) As solid support 3' phosphate CPG ( GlenResearch 20-2900-01) was used. Removal of the oligonucleotides from the solid support and deprotection was carried out with 33 % NH₃ for 8h at 55 °C. The solution was evaporated under vacuum. The remainder was dissolved in double distilled water. The labeled oligonucleotide was purified by reversed phase chromatography using a Oligo R3 4.6 x 50 mm column) Chromatography: buffer A: 0.1M Triethylammoniamacetat in water pH 7.0 buffer B: 0.1 M triethylammonium acetate in water/MeCN 1:1. gradient 2 min 0 % B in 45 min to 100 %B ( the gradient was stopped when a product starts to eluate) The product was eluated at approx. 50 % B. flow rate was 1 ml/min detection at 260 nm. The fractions from the labeled oligonucleotide peak were collected and the solvent was removed by using a vacuum centrifuge. The remainder was dissolved in double distilled water and then evaporated again with vacuum centrifuge. This procedure was repeated three times. The pellet was dissolved in water and lyophilized.

For preparation of a 3' LC Red 640 labeled oligonucleotide, synthesis was performed in the 1 µmol range. Commercially available standard phosphoramidites ( DMTr ibu G, DMTr bzA; DMTr bz C and DMTr T) and chemicals for standard synthesis were obtained from Glen Research. A 3' amino group was introduced by using commercially available 3' aminomodifier CPG (ChemGenes cat no. NSS-0004A02CL) as solid support. Removal of the aminomodified oligonucleotides from the solid support and deprotection was carried out with 33 % NH₃ for 8h at 55 °C. The solution was evaporated under vacuum. The labeling and purification was performed according to the procedure described in the packinsert of the commercially available LC Red 640 NHS ester (Roche Applied Science cat no 2015161).

3' Fluorescein labeled oligonucleotides were synthesized and purified according to the pack insert of the commercially available LightCycler Fluorescein CPG (Roche Applied Science cat no. 3138178). 5' LightCycler Red 640 labeled oligonucleotides were synthesized and purified according to the pack insert of the commercially available LightCycler Red 640 NHS Ester (Roche Applied Science cat no. 2015161).

### Example 3

Quantitative Real time PCR of Factor V DNA with and without a Nitroindole-quenched FRET donor

For amplification of a Factor V DNA fragment, a 20µl Real Time PCR reaction mixture was set up as follows:

| | |
|---|---|
| 10³ or 10⁸ | copies of a plasmid containing the Factor V gene (Gene Bank Accession No: M_014335) |
| 3 mM | MgCl₂ |
| 500 nM each | primers according to SEQ ID NO: 1 and 2 |
| 200 nM each | FRET hybridization probes according to SEQ ID NO: 3 and 4 |

PCR components of LightCycler DNA Master Hybridization Probes Kit (Roche Applied Science, Cat. No. 2158825).

The probe according to SEQ ID NO: 3 was 3' terminally labeled with LC-Red-640 (Roche Applied Science) according to example 1. The probe according to SEQ ID NO: 4 was 5'terminally labeled with Fluorescein according to example 1 or Fluorescein and a Nitroindole moiety according to example 2.

Amplification was performed in a LightCycler instrument (Roche Applied Science) according to the following thermocycling protocol:

**Table 1:**

| | T[°C] | t[sec] | Ramp-rate[°C/sec] | Acquisition | Cycles |
|---|---|---|---|---|---|
| Denaturation | 95 | 30 | 20.0 | none | 1 |
| Amplification | 95 | 0 | 20.0 | none | |
| | 55 | 10 | 20.0 | single | 45 |
| | 72 | 10 | 20.0 | none | |

Real time monitoring was performed using the 2^{nd} derivative threshold method over 45 cycles by means of dividing the absolute signal values obtained in the FRET acceptor channel through the absolute signal values obtained in the FRET donor channel thus resulting in a normalization of fluorescent signaling e.g. with respect to capillary positioning effects.

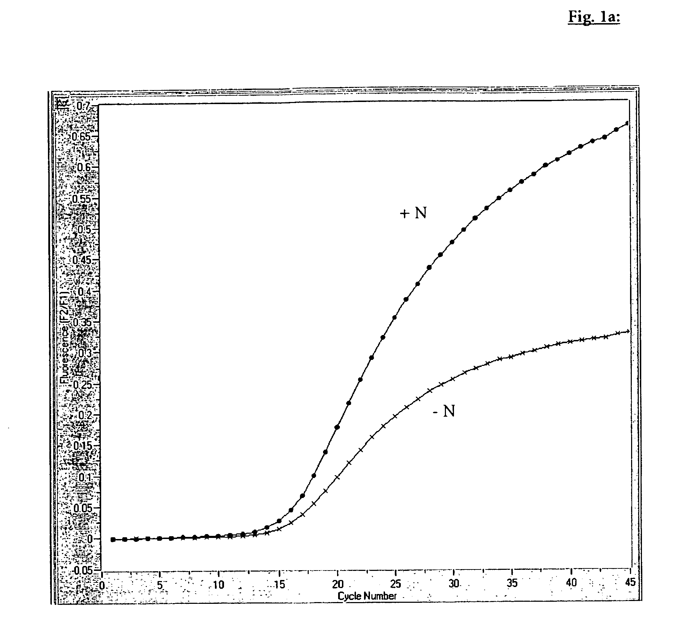

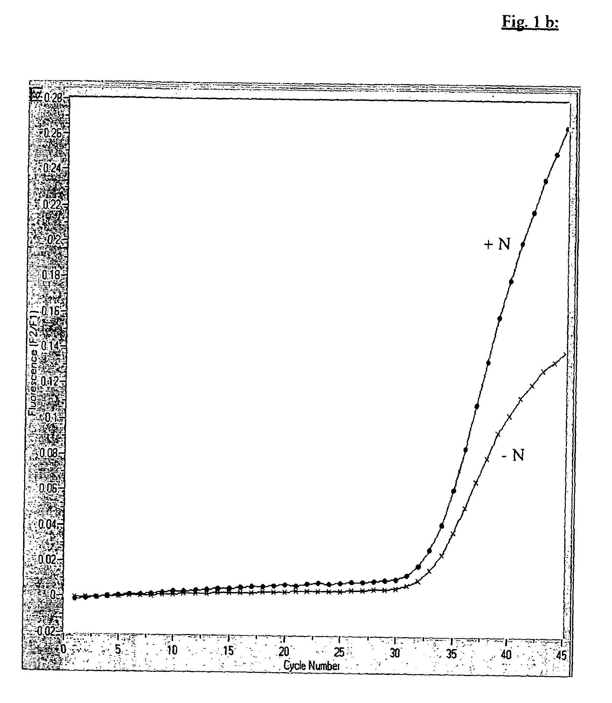

The result is shown in fig. 1. As can be seen in the figure, for both copy numbers of target DNA tested (fig. 1a: 10⁸ copies, fig. 1b: 10³ copies), usage of a FRET donor hybridization probe carrying a Nitroindole group as a quenching moiety resulted in a significantly increased amplification signal.

### Example 4

### Melting Curve Analysis of Factor V DNA with and without a Nitroindole-quenched FRET donor

Subsequent to the reaction disclosed in example 2, the samples were subjected to a melting curve analysis according the instructions of the LightCycler manual (Roche Applied Sciences) using the following temperature transition protocol:

**Table 2:**

| | T[°C] | t[sec] | Ramp-rate[°C/sec] | Acquisition | Cycles |
|---|---|---|---|---|---|
| Melting curve | 95 | 0 | 20.0 | none | |
| | 45 | 60 | 20.0 | continuous | 1 |
| | 75 | 10 | 0.1 | none | |
| Cooling | 40 | 30 | 20.0 | none | 1 |

Fluorescence monitoring was performed by means of dividing the absolute signal values obtained in channel 2 through the absolute signal values obtained in channel 1 and subsequent calculation of the first derivative.

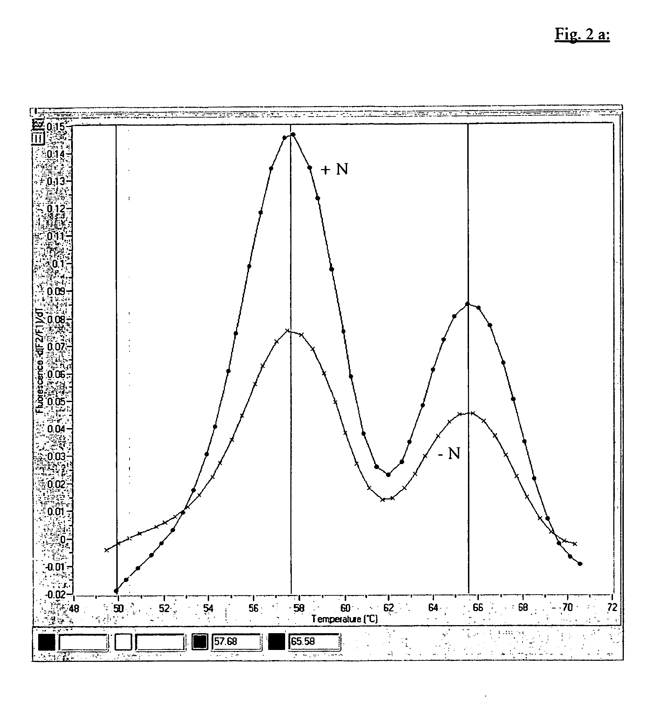

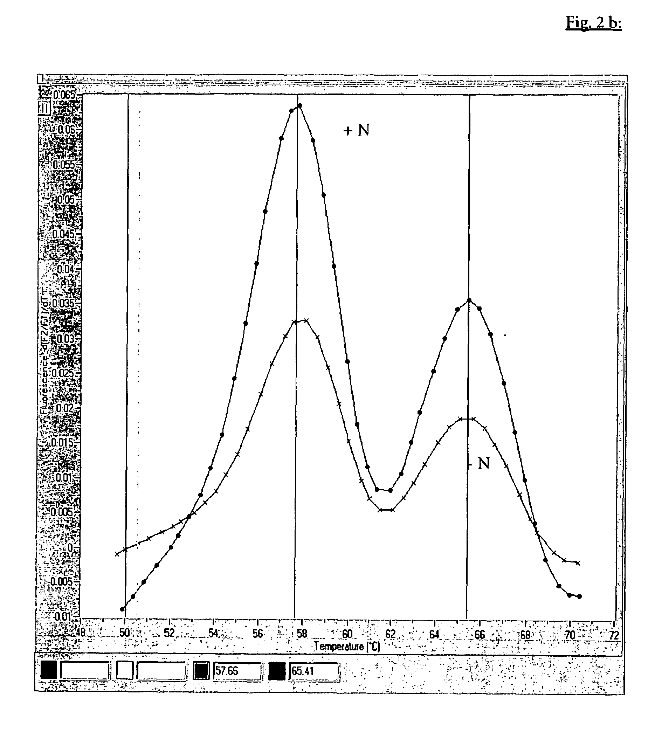

The result is shown in fig. 2. As can be seen in the figure, for both copy numbers of target DNA tested (fig. 2a: 10⁸ copies, fig. 2b: 10³ copies), usage of a FRET donor hybridization probe carrying a Nitroindole group as a quenching moiety resulted in significantly increased melting peaks.

### Example 5

### Quantitative Real time PCR of Her4 cDNA with a G quenched FRET donor

For amplification of a Her4 cDNA, two independent 20µl Real Time PCR reaction mixtures were set up basically as disclosed under example 3 with the following modifications:
- Instead of Taq Polymerase, FastStart Taq Polymerase (Roche Applied Sciences Cat. No. 2 158 264) was used.
- cDNA encoding Her4(Gene Bank Accession No: L07868), reverse transcribed from 20 ng total RNA from HeLa cells was amplified.
- 500 nM each amplification primers according to SEQ ID NO: 5 and 6
- 300 nM each FRET hybridization probes according to SEQ ID NO: 7 and 8 or, alternatively,
- 300 nM each FRET hybridization probes according to SEQ ID NO: 7 and 9

The probe according to SEQ ID NO: 7 was 5' terminally labeled with LC-Red-640 (Roche Applied Science). The probes according to SEQ ID NO: 8 and 9 differ in that the probe according to SEQ ID NO: 8 represents a hybridization probe as known in the art which is exactly complementary to the target nucleic acid, whereas the probe according to SEQ ID NO: 9 represents a hybridization probe according to the invention carrying a 3' terminal G residue which does not hybridize to the target nucleic acid. Both probes were labeled at their 3' terminal residue with Fluorescein.

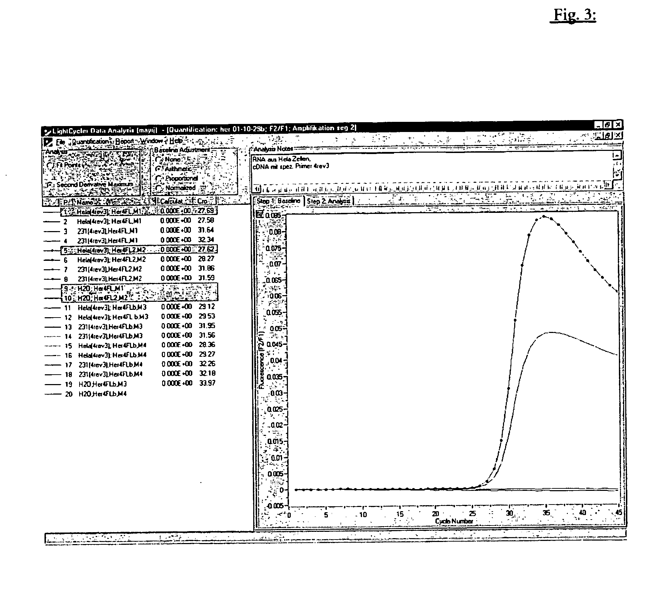

Amplification was performed basically as disclosed in example 3. The result is shown in fig. 3. The upper curve corresponds to the real time monitoring signal using a pair of FRET hybridization probes according to the invention, wherein the Fluorescein labeled probe was carrying an additional G at its 3' end according to SEQ ID NO: 9. The lower curve corresponds to a real time monitoring signal using a pair of FRET hybridization probes as it is known in the art, characterized in that a Fluorescein labeled probe according to SEQ ID NO: 8 which perfectly matches the target sequence was used. It can be concluded that the increased signal intensity using a FRET donor probe according to SEQ ID NO: 9 is due to the quenching characteristics of the additionally introduced G residue with respect to the Fluorescein compound.

### List of References

Bernard, P. S., et al., Anal Biochem 255 (1998) 101-7
Kreuzer, K. A., et al., Clin Chem 47 (2001) 486-90
Okamura, Y., et al., Nucleic Acids Res 28 (2000) E107
Seidel C.; et al., Nucleobase-Specific Quenching of Fluorescent Dyes. 1. Nucleobase One-Electron Redox Potentials and Their Correlation with Static and Dynamic Quenching Efficiencies., J. Phys. Chem. (1996), 100(13), 5541-53, CODEN: JPCHAX ISSN:0022-3654. CAN 124:261578 AN 1996:145280 CAPLUS
WO 01/86001
WO 97/46707
WO 97/46712
WO 97/46714

## Claims

1. A pair of FRET hybridization probes consisting of a first oligonucleotide carrying a FRET donor entity and a second oligonucleotide carrying a FRET acceptor entity, **characterized in that** one oligonucleotide carrying the first FRET entity is labeled at its 3' terminal residue and the second oligonucleotide is labeled at its 5'residue, said terminal residues being base paring to an adjacent residues in the target nucleic acid or to residues which are separated by only one or two further residues,
wherein the oligonucleotide carrying the donor fluorescent entity is carrying at least one second entity, said second entity being a Nitroindole which is capable of quenching fluorescence emission of said donor fluorescent entity.

2. A pair of FRET hybridization probes according to claim 1,
wherein the same nucleotide residue of said first oligonucleotide carrying the donor fluorescent entity is carrying said second entity, which is capable of quenching fluorescence emission of said donor fluorescent entity.

3. A set of at least 3 oligonucleotides, **characterized in that** a first oligonucleotide and a second oligonucleotide are capable of acting as a pair of amplification primers for a template dependent nucleic acid amplification reaction, further **characterized in that** said first oligonucleotide and a third oligonucleotide are each labeled with one corresponding member of a FRET pair consisting of a FRET donor entity and a FRET acceptor entity, and said first oligonucleotide being labeled internally with said corresponding member of a FRET pair **characterized in that** said first and said third oligonucleotide are partially complementary to each other, such that the labels of said first and third oligonucleotide are separated by no, one or two residues
wherein the oligonucleotide carrying the FRET donor entity is carrying a second entity, said second entity being a Nitroindole which is capable of quenching fluorescence emission of said donor fluorescent entity.

4. A set of oligonucleotides according to claim 3,
wherein the same nucleotide residue of said first oligonucleotide carrying the FRET donor entity is carrying said second entity which is capable of quenching fluorescence emission of said donor fluorescent entity.

5. A composition comprising a nucleic acid sample and a pair of hybridization probes according to claim 1-2 or a set of oligonucleotides according to claim 4.

6. A kit comprising a pair of hybridization probes according to claim 1-2 or a set of oligonucleotides according to claim 3-4 and at least one other component selected from a group consisting of nucleic acid amplification primers, template dependent nucleic acid polymerase, deoxynucleoside triphosphates and a buffer for template dependent nucleic acid amplification reaction.

7. Method for qualitative or quantitative detection of a nucleic acid sequence in a nucleic acid sample, wherein said nucleic acid sample is hybridized with a pair of FRET hybridization probes according to claim 1 or 2.

8. Method according to claim 7, **characterized in that** a part of said nucleic acid present in said sample which comprises a target nucleic acid sequence substantially complementary to the sequence of said hybridization probe according to claim 1 or 2 is amplified by template dependent nucleic acid amplification reaction, preferably a polymerase chain reaction.

9. Method for qualitative or quantitative detection of a nucleic acid sequence in a nucleic acid sample, comprising template dependent nucleic acid amplification using a primer pair according to said first and said second oligonucleotide of claim 3 or 4, and hybridization of the amplification product with said third oligonucleotide of claim 3 or 4.

10. Method according to claims 8-9, wherein fluorescence emission of either the donor fluorescent entity or emission of the acceptor fluorescent entity is monitored in Real Time.

11. Method according to claim 10, **characterized in that** fluorescence emission of said FRET donor entity is monitored in a first detector channel and fluorescence emission of said FRET acceptor entity is monitored in a second detector channel, further **characterized in that** the fluorescence emission of said FRET acceptor entity is normalized by the fluorescence emission of said FRET donor entity.

12. Method for the determination of the melting profile of a hybrid consisting of a target nucleic acid and a pair of FRET hybridization probes according to claim 1-2, **characterized in that** the fluorescence emission is determined as a function of temperature.

13. Method according to claims 12, **characterized in that** fluorescence emission of the FRET donor entity is monitored in a first detector channel and fluorescence emission of the FRET acceptor entity is monitored in a second detector channel, further **characterized in that** the fluorescence emission of said FRET acceptor entity is normalized by the fluorescence emission of said FRET donor entity.

## Patentansprüche

1. FRET-Hybridisierungssondenpaar, bestehend aus einem eine FRET-Donoreinheit tragenden ersten Oligonukleotid und einem eine FRET-Akzeptoreinheit tragenden zweiten Oligonukleotid, **dadurch gekennzeichnet, daß** ein Oligonukleotid, das die erste FRET-Einheit trägt, an seinem 3'-terminalen Rest und das zweite Oligonukleotid an seinem 5'-Rest markiert ist, wobei die terminalen Reste eine Basenpaarung mit einem benachbarten Rest in der Zielnukleinsäure oder mit Resten, die nur durch einen oder zwei weitere Reste getrennt sind, eingehen,
wobei das die Donorfluoreszenzeinheit tragende Oligonukleotid wenigstens eine zweite Einheit trägt, wobei es sich bei der zweiten Einheit um ein Nitroindol handelt, das zum Quenchen der Fluoreszenzemission der Donorfluoreszenzeinheit fähig ist.

2. FRET-Hybridisierungssondenpaar nach Anspruch 1,
wobei der gleiche Nukleotidrest des die Donorfluoreszenzeinheit tragenden Oligonukleotids die zweite Einheit trägt, die zum Quenchen der Fluoreszenzemission der Donorfluoreszenzeinheit fähig ist.

3. Satz von wenigstens 3 Oligonukleotiden, **dadurch gekennzeichnet, daß** ein erstes Oligonukleotid und ein zweites Oligonukleotid dazu in der Lage sind, als ein Amplifikationsprimerpaar für eine matrizenabhängige Nukleinsäureamplifikationsreaktion zu fungieren, ferner **dadurch gekennzeichnet, daß** das erste Oligonukleotid sowie ein drittes Oligonukleotid jeweils mit einem entsprechenden Mitglied eines aus einer FRET-Donoreinheit und einer FRET-Akzeptoreinheit bestehenden FRET-Paars markiert ist und das erste Oligonukleotid intern mit dem entsprechenden Mitglied eines FRET-Paars markiert ist, **dadurch gekennzeichnet, daß** das erste und das dritte Oligonukleotid teilweise komplementär zueinander sind, so daß die Markierungen des ersten und dritten Oligonukleotids durch keinen, einen oder zwei Reste getrennt sind,
wobei das die FRET-Donoreinheit tragende Oligonukleotid eine zweite Einheit trägt, wobei es sich bei der zweiten Einheit um ein Nitroindol handelt, das zum Quenchen der Fluoreszenzemission der Donorfluoreszenzeinheit fähig ist.

4. Satz von Oligonukleotiden nach Anspruch 3,
wobei der gleiche Nukleotidrest des die FRET-Donoreinheit tragenden Oligonukleotids die zweite Einheit trägt, die zum Quenchen der Fluoreszenzemission der Donorfluoreszenzeinheit fähig ist.

5. Zusammensetzung, umfassend eine Nukleinsäureprobe sowie ein Hybridisierungssondenpaar nach Anspruch 1-2 oder einen Satz von Oligonukleotiden nach Anspruch 4.

6. Kit, umfassend ein Hybridisierungssondenpaar nach Anspruch 1-2 oder einen Satz von Oligonukleotiden nach Anspruch 3-4 sowie wenigstens eine weitere Komponente, ausgewählt aus einer Gruppe bestehend aus Nukleinsäureamplifikationsprimern, matrizenabhängiger Nukleinsäurepolymerase, Desoxynukleosidtriphosphaten und einem Puffer für die matrizenabhängige Nukleinsäureamplifikationsreaktion.

7. Verfahren zum qualitativen und quantitativen Nachweis einer Nukleinsäuresequenz in einer Nukleinsäureprobe, wobei die Nukleinsäureprobe mit einem FRET-Hybridisierungssondenpaar nach Anspruch 1 oder 2 hybridisiert wird.

8. Verfahren nach Anspruch 7, **dadurch gekennzeichnet, daß** ein Teil der in der Probe vorhandenen Nukleinsäure, der eine zur Sequenz der Hybridisierungssonde nach Anspruch 1 oder 2 weitgehend komplementäre Zielnukleinsäuresequenz umfaßt, durch eine matrizenabhängige Nukleinsäureamplifikationsreaktion, vorzugsweise eine Polymerasekettenreaktion, amplifiziert wird.

9. Verfahren zum qualitativen und quantitativen Nachweis einer Nukleinsäuresequenz in einer Nukleinsäureprobe, umfassend die matrizenabhängige Nukleinsäureamplifikationsreaktion unter Verwendung eines Primerpaars gemäß des ersten und des zweiten Oligonukleotids aus Anspruch 3 oder 4 sowie die Hybridisierung des Amplifikationsprodukts mit dem dritten Oligonukleotid aus Anspruch 3 oder 4.

10. Verfahren nach Ansprüchen 8-9, wobei die Fluoreszenzemission entweder der Donorfluoreszenzeinheit oder die Emission der Akzeptorfluoreszenzeinheit in Echtzeit verfolgt wird.

11. Verfahren nach Anspruch 10, **dadurch gekennzeichnet, daß** die Fluoreszenzemission der FRET-Donoreinheit in einem ersten Detektorkanal und die Fluoreszenzemission der FRET-Akzeptoreinheit in einem zweiten Detektorkanal verfolgt wird, ferner **dadurch gekennzeichnet, daß** die Fluoreszenzemission der FRET-Akzeptoreinheit über die Fluoreszenzemission der FRET-Donoreinheit normiert wird.

12. Verfahren zur Bestimmung des Schmelzprofils eines aus einer Zielnukleinsäure und einem FRET-Hybridisierungssondenpaar nach Anspruch 1-2 bestehenden Hybrids, **dadurch gekennzeichnet, daß** die Fluoreszenzemission in Abhängigkeit von der Temperatur bestimmt wird.

13. Verfahren nach Anspruch 12, **dadurch gekennzeichnet, daß** die Fluoreszenzemission der FRET-Donoreinheit in einem ersten Detektorkanal und die Fluoreszenzemission der FRET-Akzeptoreinheit in einem zweiten Detektorkanal verfolgt wird, ferner **dadurch gekennzeichnet, daß** die Fluoreszenzemission der FRET-Akzeptoreinheit über die Fluoreszenzemission der FRET-Donoreinheit normiert wird.

## Revendications

1. Paire de sondes d'hybridation FRET se composant d'un premier oligonucléotide portant une entité de donneur FRET et un deuxième oligonucléotide portant une entité d'accepteur FRET, **caractérisée en ce qu'**un oligonucléotide portant la première entité FRET est marqué au niveau de son résidu terminal 3' et le deuxième oligonucléotide est marqué au niveau de son résidu 5', lesdits résidus terminaux étant appariés par des bases à des résidus adjacents dans l'acide nucléique cible, ou à des résidus qui ne sont séparés que par un ou deux résidus supplémentaires, dans laquelle l'oligonucléotide portant l'entité fluorescente de donneur porte au moins une deuxième entité, ladite deuxième entité étant un groupe nitroindole qui est capable d'éteindre l'émission de fluorescence de ladite entité fluorescente de donneur.

2. Paire de sondes d'hybridation FRET selon la revendication 1, dans laquelle le même résidu nucléotide dudit premier oligonucléotide portant l'entité fluorescente de donneur porte ladite deuxième entité, qui est capable d'éteindre l'émission de fluorescence de ladite entité fluorescente de donneur.

3. Ensemble d'au moins 3 oligonucléotides, **caractérisé en ce qu'**un premier oligonucléotide et un deuxième oligonucléotide sont capables de faire office de paire d'amorceurs d'amplification pour une réaction d'amplification d'acide nucléique dépendante d'une matrice, **caractérisé en outre en ce que** ledit premier oligonucléotide et un troisième oligonucléotide sont marqués chacun par un élément correspondant d'une paire FRET se composant d'une entité de donneur FRET et d'une entité d'accepteur FRET, et ledit premier oligonucléotide étant marqué de façon interne par ledit élément correspondant d'une paire FRET, **caractérisé en ce que** ledit premier oligonucléotide et ledit troisième oligonucléotide sont partiellement complémentaires l'un à l'autre, de sorte que les marques desdits premier et troisième oligonucléotides sont séparés par aucun résidu, un ou deux résidus, dans lequel l'oligonucléotide portant l'entité de donneur FRET porte une deuxième entité, ladite deuxième entité étant un groupe nitroindole qui est capable d'éteindre l'émission d e fluorescence de ladite entité fluorescente de donneur.

4. Ensemble d'oligonucléotides selon la revendication 3,
dans lequel le même résidu nucléotide dudit premier oligonucléotide portant l'entité de donneur FRET porte ladite deuxième entité qui est capable d'éteindre l'émission de fluorescence de ladite entité fluorescente de donneur.

5. Composition comprenant un échantillon d'acide nucléique et une paire de sondes d'hybridation selon la revendication 1-2 ou un ensemble d'oligonucléotides selon la revendication 4.

6. Nécessaire comprenant une paire de sondes d'hybridation selon la revendication 1-2 ou un ensemble d'oligonucléotides selon la revendication 3-4, et au moins un autre composant choisi dans le groupe formé par les amorceurs d'amplification d'acide nucléique, la polymérase d'acide nucléique dépendant d'une matrice, les triphosphates de désoxynucléoside et un tampon pour une réaction d'amplification d'acide nucléique dépendant d'une matrice.

7. Procédé pour la détection qualitative ou quantitative d'une séquence d'acide nucléique dans un échantillon d'acide nucléique, dans lequel ledit échantillon d'acide nucléique est hybridé avec une paire de sondes d'hybridation FRET selon la revendication 1 ou 2.

8. Procédé selon la revendication 7, **caractérisé en ce qu'**une partie dudit acide nucléique présent dans ledit échantillon qui comprend une séquence d'acide nucléique cible sensiblement complémentaire à la séquence de ladite sonde d'hybridation selon la revendication 1 ou 2, est amplifiée par la réaction d'amplification d'acide nucléique dépendant d'une matrice, de préférence une amplification en chaîne par polymérase.

9. Procédé pour la détection qualitative ou quantitative d'une séquence d'acide nucléique dans un échantillon d'acide nucléique, comprenant une amplification d'acide nucléique dépendant d'une matrice en employant une paire d'amorces selon ledit premier oligonucléotide et ledit deuxième oligonucléotide selon la revendication 3 o u 4, et l'hybridation du produit d'amplification avec ledit troisième oligonucléotide selon la revendication 3 ou 4.

10. Procédé selon les revendications 8-9, dans lequel l'émission de fluorescence de l'entité fluorescente de donneur ou l'émission d'entité fluorescente d'accepteur sont contrôlées en temps réel.

11. Procédé selon la revendication 10, **caractérisé en ce que** l'émission de fluorescence de ladite entité de donneur FRET est contrôlée dans un premier canal de détecteur, et l'émission de fluorescence de ladite entité d'accepteur FRET est contrôlée dans un deuxième canal de détecteur, **caractérisé en outre en ce que** l'émission de fluorescence de ladite entité d'accepteur FRET est normalisée par l'émission de fluorescence de ladite entité de donneur FRET.

12. Procédé pour déterminer le profil de fusion d'un hybride se composant d'un acide nucléique cible et d'une paire de sondes d'hybridation FRET selon la revendication 1-2, **caractérisé en ce que** l'émission de fluorescence est déterminée en fonction de la température.

13. Procédé selon la revendication 12, **caractérisé en ce que** l'émission de fluorescence de l'entité de donneur FRET est contrôlée dans un premier canal de détecteur, et l'émission de fluorescence de l'entité d'accepteur FRET est contrôlée dans un deuxième canal de détecteur, **caractérisé en outre en ce que** l'émission de fluorescence de ladite entité d'accepteur FRET est normalisée par l'émission de fluorescence de ladite entité d e donneur FRET.
